# EUROPÄISCHE PATENTSCHRIFT

(11) **EP 2 010 235 B1**
(45) Veröffentlichungstag und Bekanntmachung des Hinweises auf die Patenterteilung: **15.01.2014**
(21) Anmeldenummer: 07723745.1
(22) Anmeldetag: 29.03.2007
(51) Int. Cl.: A61L 15/26, C08G 18/08, C08G 18/12, C08G 18/28, C08G 18/40, C08G 18/44, C08G 18/48, C08G 18/72, C08J 9/30

(54) **VERFAHREN ZUR HERSTELLUNG VON POLYURETHAN-SCHÄUMEN**
METHOD FOR PRODUCTION OF POLYURETHANE FOAMS
PROCEDE DE FABRICATION DE MOUSSES DE POLYURETHANNE

(30) Priorität: 08.04.2006 DE 102006016639
(43) Veröffentlichungstag der Anmeldung: 07.01.2009
(73) Patentinhaber: Bayer MaterialScience AG, 51368 Leverkusen (DE)
(72) Erfinder: MAGER, Michael, 51375 Leverkusen (DE); RISCHE, Thorsten, 59423 Unna (DE); DÖRR, Sebastian, 40597 Düsseldorf (DE); FELLER, Thomas, 42659 Solingen (DE); HECKES, Michael, 47800 Krefeld (DE); DIETZE, Melita, 40699 Erkrath (DE); FUGMANN, Burkhard, 40878 Ratingen (DE)
(86) Internationale Anmeldenummer: PCT/EP2007/002801
(87) Internationale Veröffentlichungsnummer: WO 2007/115697

(56) Entgegenhaltungen:
- WO-A-00/74739
- WO-A-02/090413
- DE-A1- 4 418 319
- DE-A1- 19 804 665
- US-A1- 2004 138 319
- US-A1- 2005 037 058

## Beschreibung

Die Erfindung betrifft ein Verfahrung zur Herstellung von Polyurethan-Schäumen, durch Aufschäumen und Trocknen spezieller Polyurethan-Dispersionen.

In der Wundbehandlung ist die Verwendung von Polyurethan-Schäumen als Wundauflage seit langem bekannt. Um eine gute Absorption von Wundflüssigkeit zu gewährleisten, werden dazu in der Regel hydrophile Polyurethan-Schäume eingesetzt. Diese werden erhalten durch Umsetzung von Mischungen aus Diisocyanaten und Polyolen bzw. NCO-funktionellen Polyurethan-Prepolymeren mit Wasser in Gegenwart bestimmter Katalysatoren sowie (Schaum-) Additiven. In der Regel werden hierbei aromatische Diisocyanate eingesetzt, da sich diese am besten verschäumen lassen. Zahlreiche Ausführungsformen dieser Verfahren sind bekannt, beispielsweise beschrieben in US 3,978,266, US 3,975,567 und EP-A 0 059 048. Die vorgenannten Verfahren weisen jedoch den Nachteil auf, dass reaktive Mischungen eingesetzt werden müssen, enthaltend Diisocyanate oder entsprechende Prepolymere, deren Handhabung technisch aufwendig ist, da z.B. entsprechende Schutzmaßnahmen erforderlich sind.

Es ist darüber hinaus bekannt, Schäume aus Polyurethan-Dispersionen herzustellen, indem man in Gegenwart geeigneter (Schaum-) Additive durch kräftiges Rühren Luft einträgt. Nach dem Trocknen und Aushärten werden so genannte mechanische Polyurethan-Schäume erhalten. Solche Schäume sind im Zusammenhang mit Wundauflagen in EP-A 0 235 949 und EP-A 0 246 723 beschrieben, wobei dem Schaum entweder ein selbst-haftendes Polymer zugesetzt oder der Schaum auf einen Film eines selbst-haftenden Polymers aufgebracht wird. Die Verwendung der Schäume als solche, d.h. ohne selbst-haftende Polymere wird nicht beschrieben. Die in EP 0 235 949 und EP 0 246 723 aufgeführten Beispiele beschreiben darüber hinaus zwingend die Verwendung von Polyaziridinen als Vernetzer, die nach heutigem Stand des Wissens aufgrund ihrer Toxizität nicht mehr akzeptabel sind. Zur Vernetzung müssen überdies hohe Einbrenntemperaturen angewandt werden, angegeben sind 100 °C bis 170 °C. In US 4,655,210 ist die Verwendung der vorgenannten mechanischen Schäume für Wundauflagen beschrieben, die einen speziellen Aufbau aus Träger, Schaum und Hautkontakt-Schicht aufweisen.

Die in EP-A 0 235 949, EP-A 0 246 723 und US 4,655,210 beschriebenen Polyurethan-Dispersionen werden durch den Einbau von bestimmten Carbonsäuren wie Dimethylolcarbonsäuren und Neutralisation der Carbonsäuren mit tertiären Aminen, beispielsweise Triethylamin, anionisch hydrophiliert. Die so gebildeten Ammoniumcarboxylate sind jedoch zersetzlich, insbesondere bei höheren Temperaturen, wodurch die Amine wieder freigesetzt werden. Dies ist bei der Verarbeitung solcher Produkte und besonders in Hautkontakt von großem Nachteil. Bei der Herstellung dieser Polyurethan-Dispersionen wurden die Dimethylolcarbonsäuren darüber hinaus gelöst eingesetzt, z.B. in Dimethylformamid oder N-Methylpyrrolidon, wodurch die fertigen Produkte insgesamt einen hohen Gehalt an gesundheitlich bedenklichen, flüchtigen organischen Bestandteilen (VOC) aufweisen, im Falle des eingesetzten Witcobond™ 290 H 10,8 g/Liter (ohne Wasser).

EP-A 0 760 743 beschreibt darüber hinaus solche mechanischen Schäume auf Basis von Latex-Dispersionen, die jedoch nicht aus Polyurethanen bestehen und schlechtere mechanische Eigenschaften aufweisen.

Aufgabe der vorliegenden Erfindung war daher die Bereitstellung von neuen Wundauflagen auf Basis von Polyurethanen, welche auf möglichst einfache Weise und ohne die Verwendung gesundheitsschädlicher Aufbaukomponenten oder Additive zugänglich sind. Weitere Voraussetzung ist, dass diese Wundauflagen gute mechanische Eigenschaften, eine hohe Aufnahmekapazität von physiologischer Salzlösung sowie eine hohe Wasserdampfdurchlässigkeit aufweisen.

Es wurde nun gefunden, dass solche Wundauflagen auf Polyurethanbasis zugänglich sind, indem Zusammensetzungen, die spezielle wässrige Polyurethan-Dispersionen enthalten, aufgeschäumt und anschließend physikalisch getrocknet werden.

Gegenstand der vorliegenden Erfindung ist daher ein Verfahren zur Herstellung von Wundauflagen, bei dem Zusammensetzungen enthaltend anionisch hydrophilierte, wässrige Polyurethan-Dispersionen (I) aufgeschäumt und physikalisch ohne chemische Vernetzung getrocknet werden, dadurch gekennzeichnet, dass die Polyurethan-Dispersionen (I) zur anionischen Hydrophilierung ausschließlich Sulfonatgruppen enthalten und die Dispersionen (I) Feststoffgehalte von 55 bis 65 Gew.-% bezogen auf das darin enthaltene Polyurethan aufweisen. Unter Vernetzung wird im Sinne der vorliegenden Erfindung die Bildung kovalenter Bindungen verstanden.

Wundauflagen aus Polyurethan-Schäumen im Sinne der Erfindung sind poröse Materialien, bevorzugt mit zumindest teilweise vorhandener Offenzelligkeit, welche im wesentlichen aus Polyurethanen bestehen und Wunden im Sinne einer sterilen Abdeckung vor Keimen bzw. Umgebungseinflüssen schützen, eine schnelle und hohe Absorption von physiologischer Salzlösung bzw. Wundflüssigkeit aufweisen, durch geeignete Feuchtdurchlässigkeit für ein geeignetes Wundklima sorgen und eine ausreichende mechanische Festigkeit aufweisen.

Die speziellen Polyurethan-Dispersionen (I) sind anionisch mittels Sulfonatgruppen hydrophiliert. Bevorzugt sind zur anionischen Hydrophilierung ausschließlich Sulfonatgruppen enthalten.

Bevorzugt weisen die speziellen Polyurethan-Dispersionen (I) einen niedrigen Grad an hydrophilen anionischen Gruppen, bevorzugt von 0,1 bis 15 Milliequivalenten pro 100g Polyurethan (Festharz) auf.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 500 nm, bestimmt mittels Laserkorrelations-Spektroskopie.

Bevorzugt besitzen die Polyurethan-Dispersionen (I) Feststoffgehalte von 55 bis 65 Gew.-% und besonders bevorzugt 60 bis 65 Gew.-%, bezogen auf das darin enthaltene Polyurethan.

Bevorzugt weisen diese Polyurethan-Dispersionen weniger als 0,5 Gew.-%, besonders bevorzugt weniger als 0,2 Gew.-%, bezogen auf die gesamte Dispersionen, an ungebundenen organischen Aminen auf.

Solche bevorzugt einzusetzenden Polyurethan-Dispersionen (I) sind erhältlich, in dem
A) isocyanatfunktionelle Prepolymere aus
   A1) organischen Polyisocyanaten
   A2) polymeren Polyolen mit zahlenmittleren Molekulargewichten von 400 bis 8000 g/mol, vorzugsweise 400 bis 6000 g/mol und besonders bevorzugt von 600 bis 3000 g/mol, und OH-Funktionalitäten von 1,5 bis 6, bevorzugt 1,8 bis 3, besonders bevorzugt von 1,9 bis 2,1, und
   A3) gegebenenfalls hydroxyfunktionellen Verbindungen mit Molekulargewichten von 62 bis 399 g/mol sowie
   A4) gegebenenfalls isocyanatreaktiven, anionischen oder potentiell anionischen und/oder gegebenenfalls nichtionischen Hydrophilierungsmitteln,
   hergestellt, und
B) dessen freie NCO-Gruppen dann ganz oder teilweise
   B1) gegebenenfalls mit aminofunktionellen Verbindungen mit Molekulargewichten von 32 bis 400 g/mol und
   B2) mit aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden.

Falls gewünscht, kann das Prepolymer vor, während oder nach der Dispergierung durch Beimischung einer Base ganz oder teilweise in die anionische Form überführt werden.

Um eine anionische Hydrophilierung zu erreichen müssen in A4) und/oder B2) Hydrophilierungsmittel eingesetzt werden, die wenigstens eine gegenüber NCO-Gruppen reaktive Gruppe wie Amino-, Hydroxy- oder Thiolgruppen aufweisen und darüber hinaus -COO- oder - SO₃⁻ oder -PO₃²⁻ als anionische bzw. deren ganz oder teilweise protonierte Säureformen als potentiell anionische Gruppen aufweisen.

Bevorzugt werden in A4) und/oder B2) solche Verbindungen zur anionischen oder potentiell anionischen Hydrophilierung eingesetzt, die als anionische oder potentiell anionische Funktionalität ausschließlich Sulfonsäure- bzw. Sulfonatgruppen (-SO₃H bzw. -SO₃M, mit M = Alkali- oder Erdalkalimetall) aufweisen.

Geeignete Polyisocyanate der Komponente A1) sind die dem Fachmann an sich bekannten aliphatischen oder cycloaliphatischen Polyisocyanate einer NCO-Funktionalität von größer oder gleich 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis-(4,4`-isocyanatocyclohexyl)methane oder deren Mischungen beliebigen Isomerengehalts, 1,4-Cyclohexylendiisocyanat, 4-Isocyanatomethyl-1,8-octandiisocyanat (Nonantriisocyanat), sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate die eine Funktionalität ≥ 2 aufweisen, mit Uretdion-, Isocyanurat-, Urethan-, Allophanat-, Biuret-, Iminooxadiazindion- oder Oxadiazintrionstruktur sowie Mischungen aus diesen anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocyanatgruppen oder Mischungen aus diesen und einer mittleren NCO-Funktionalität der Mischung von 2 bis 4, bevorzugt 2 bis 2,6 und besonders bevorzugt 2 bis 2,4.

Besonders bevorzugt werden in A1) Hexamethylendiisocyanat, Isophorondiisocyanat oder die isomeren Bis-(4,4'-isocyanatocyclohexyl)methane sowie Mischungen der vorgenannten Diisocyanate eingesetzt.

In A2) werden polymere Polyole mit einem zahlenmittleren Molekulargewicht Mₙ von 400 bis 8000 g/mol, bevorzugt von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Solche Polyesterpolyole sind die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in A2) hydroxylgruppenaufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art in Frage.

Bevorzugt enthält die Diolkomponente 40 bis 100 Gew.-% Hexandiol, bevorzugt sind 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Hydroxylgruppen aufweisende Polycarbonate sind bevorzugt linear gebaut.

Ebenfalls können in A2) Polyetherpolyole eingesetzt werden.

Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxid und/oder Epichlorhydrin an di- oder polyfunktionelle Startermoleküle. Polyetherpolyole, basierend auf der zumindest anteiligen Addition von Ethylenoxid an di- oder polyfunktionelle Startermoleküle, können auch als Komponente A4) eingesetzt werden (nichtionische Hydrophilierungsmittel).

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

In A3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-ε-hydroxy-capronsäureester, ω-Hydroxyhexyl-γ-hydroxybuttersäure-ester, Adipinsäure-(ß-hydroxyethyl)ester oder Terephthalsäurebis(ß-hydroxyethyl)-ester.

Ferner können in A3) auch monofunktionelle isocyanatreaktive Hydroxyl-gruppenhaltige Verbindungen eingesetzt werden. Beispiele solcher monofunktionellen Verbindungen sind Ethanol, n-Butanol, Ethylenglykolmonobutylether, Diethylenglykolmonomethylether, Diethylenglykolmonobutylether, Propylenglykolmonomethylether, Dipropylenglykolmonomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Geeignete anionisch hydrophilierende Verbindungen der Komponente A4) sind Salze der Mono- und Dihydroxysulfonsäuren. Beispiele solcher anionischen Hydrophilierungsmittel sind das Addukt von Natriumbisulfit an Buten-2-diol-1,4, wie es in DE-A 2 446 440, Seite 5 - 9, Formel I-III beschrieben ist.

Geeignete nichtionisch hydrophilierende Verbindungen der Komponente A4) sind z.B. Polyoxyalkylenether, die mindestens eine Hydroxy-, Amino- oder Thiolgruppe enthalten. Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Als Komponente B1) können organische Di- oder Polyamine wie beispielsweise 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 4,4-Diaminodicyclohexylmethan und/oder Dimethylethylendiamin eingesetzt werden.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-amine-1-ethylaminopropan, 3-Amino-1-cyclohexylaminopropan, 3-Amino-1- methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)-aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin.

Geeignete anionisch hydrophilierende Verbindungen der Komponente B2) sind Alkalimetallsalze der Mono- und Diaminosulfonsäuren. Beispiele solcher anionischen Hydrophilierungsmittel sind Salze der 2-(2-Aminoethylamino)ethansulfonsäure, Ethylendiaminpropyl- oder -butylsulfonsäure, 1,2- oder 1,3-Propylendiamin-ß-ethylsulfonsäure oder Taurin. Weiterhin kann das Salz der Cyclohexylaminopropansulfonsäure (CAPS) aus WO-A 01/88006 als anionisches Hydrophilierungsmittel verwendet werden.

Besonders bevorzugte anionische Hydrophilierungsmittel B2) sind solche, die Sulfonatgruppen als ionische Gruppen und zwei Aminogruppen enthalten, wie die Salze der 2-(2-Aminoethylamino)ethylsulfonsäure und 1,3-Propylendiamin-β-ethylsulfonsäure.

Zur Hydrophilierung können auch Mischungen aus anionischen und nichtionischen Hydrophilierungsmitteln verwendet werden.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.-% A2),
0,5 bis 20 Gew.-% Summe der Komponenten A3) und B1)
0,1 bis 25 Gew.-% Summe der Komponenten A4) und B2), wobei bezogen auf die Gesamtmengen der Komponenten A1) bis A4) und B1) bis B2) 0,1 bis 5 Gew.-% an anionischen bzw. potentiell anionischen Hydrophilierungsmitteln aus A4) und/oder B2) verwendet werden.

In einer besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.-% Komponente A1),
60 bis 90 Gew.-% A2),
0,5 bis 15 Gew.-% Summe der Komponenten A3) und B1)
0,1 bis 15 Gew.-% Summe der Komponenten Komponente A4) und B2), wobei bezogen auf die Gesamtmengen der Komponenten A1) bis A4) und B1) bis B2) 0,2 bis 4 Gew.-% an anionischen bzw. potentiell anionischen Hydrophilierungsmitteln aus A4) und/oder B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1),
65 bis 85 Gew.-% A2),
0,5 bis 14 Gew.-% Summe der Komponenten A3) und B1)
0,1 bis 13,5 Gew.-% Summe der Komponenten A4) und B2), wobei bezogen auf die Gesamtmengen der Komponenten A1) bis A4) und B1) bis B2) 0,5 bis 3,0 Gew.-% an anionischen bzw. potentiell anionischen Hydrophilierungsmitteln aus A4) und/oder B2) verwendet werden.

Die Herstellung der speziellen Polyurethan-Dispersionen kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z. B. Prepolymer-Mischverfahren, Acetonverfahren oder Schmelzdispergierverfahren verwendet werden. Bevorzugt wird nach dem Aceton-Verfahren verfahren.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanat-Gruppen zu isocyanatreaktiven Gruppen 1,05 bis 3,5, bevorzugt 1,1 bis 3,0, besonders bevorzugt 1,1 bis 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Anschluss wird in einem weiteren Verfahrensschritt, falls noch nicht oder nur teilweise geschehen, das erhaltene Prepolymer mit Hilfe von aliphatischen Ketonen wie Aceton oder 2-Butanon gelöst.

Bei der Kettenverlängerung in Stufe B) werden NH₂- und/oder NH-funktionelle Komponenten mit den noch verbliebenen Isocyanatgruppen des Prepolymers umgesetzt. Bevorzugt wird die Kettenverlängerung/-terminierung vor der Dispergierung in Wasser durchgeführt.

Geeignete Komponenten zur Kettenverlängerung sind organische Di- oder Polyamine B1) wie beispielsweise Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Diaminodicyclohexylmethan und/oder Dimethylethylendiamin.

Darüber hinaus können auch Verbindungen B1), die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1-methylaminobutan, Alkanolamine wie N-Aminoethylethanolamin, Ethanolamin, 3-Aminopropanol, Neopentanolamin zur Kettenverlängerung bzw. -terminierung eingesetzt werden.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin verwendet.

Werden zur Kettenverlängerung anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Der Kettenverlängerungsgrad, also das Äquivalentverhältnis von NCO-reaktiven Gruppen der zur Kettenverlängerung und Kettenterminierung eingesetzten Verbindungen zu freien NCO-Gruppen des Prepolymers, liegt zwischen 40 und 150 %, bevorzugt zwischen 50 und 120 %, besonders bevorzugt zwischen 60 und 120 %.

Die aminischen Komponenten B1) und B2), können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den erfindungswesentlichen Dispersionen beträgt typischerweise weniger als 1 Gew.-%, bevorzugt weniger als 0,5 Gew.-% bezogen auf die gesamte Dispersion.

Der pH-Wert der erfindungswesentlichen Dispersionen beträgt typischerweise weniger als 8,0, bevorzugt weniger als 7,5 und liegt besonders bevorzugt zwischen 5,5 und 7,5.

Neben den Dispersionen (I) können die aufzuschäumenden Zusammensetzungen auch Hilfs- und Zusatzstoffe (II) enthalten.

Beispiele für solche Hilfs- und Zusatzstoffe (II) sind Schaumhilfsmittel wie Schaumbildner und -stabilisatoren, Verdicker bzw. Thixotropiermittel, Antioxidantien, Lichtschutzmittel, Emulgatoren, Weichmacher, Pigmente, Füllstoffe und Verlaufshilfsmittel.

Bevorzugt sind als Hilfs- und Zusatzstoffe (II) Schaumhilfsmittel wie Schaumbildner und - stabilisatoren enthalten. Geeignet sind handelsübliche Verbindungen wie Fettsäureamide, Kohlenwasserstoffsulfonate, -sulfate oder Fettsäuresalze, wobei der lipophile Rest bevorzugt 12 bis 24 Kohlenstoffatome enthält, sowie Alkylpolyglycoside, die nach dem Fachmann an sich bekannten Methoden durch Umsetzung von längerkettigen Monoalkoholen (4 bis 22 C-Atome im Alkylrest), mit Mono-, Di- oder Polysacchariden erhältlich sind (siehe z.B. Kirk-Othmer, Encyclopedia of Chemical Technology, John Wiley & Sons, Vol. 24, S. 29).

Bevorzugte Schaumhilfsmittel sind Sulfosuccinamide, Alkansulfonate oder -sulfate mit 12 bis 22 Kohlenstoffatomen im Kohlenwasserstoffrest, Alkylbenzolsulfonate oder -sulfate mit 14 bis 24 Kohlenstoffatomen im Kohlenwasserstoffrest oder Fettsäureamide oder Fettsäuresalze mit 12 bis 24 Kohlenstoffatomen.

Solche Fettsäureamide sind vorzugsweise solche auf Basis von Mono- oder Di-(C2-3-alkanol)aminen. Die Fettsäuresalze können beispielsweise Alkalimetallsalze, Aminsalze oder unsubstituierte Ammoniumsalze sein.

Solche Fettsäurederivate basieren typischerweise auf Fettsäuren wie Laurinsäure, Myristinsäure, Palmitinsäure, Ölsäure, Stearinsäure, Ricinolsäure, Behensäure oder Arachidinsäure, Kokosfettsäure, Talgfettsäure, Sojafettsäure und deren Hydrierungsprodukten.

Besonders bevorzugte Schaumhilfsmittel sind Mischungen aus Sulfosuccinamiden und Ammoniumstearaten, wobei diese bevorzugt 20 bis 60 Gew.-% besonders bevorzugt 30 bis 50 Gew.-% an Ammoniumstearaten und bevorzugt 80 bis 40 Gew.-%, besonders bevorzugt 70 bis 50 Gew.-% an Sulfosuccinamiden enthalten.

Als Verdicker können handelsübliche Verdicker eingesetzt werden, wie Dextrin-, Stärke- oder Cellulosederivate, z.B. Celluloseether oder Hydroxyethylcellulose, organische vollsynthetische Verdicker auf Basis von Polyacrylsäuren, Polyvinylpyrrolidonen, Poly(meth)-acrylverbindungen oder Polyurethanen (assoziative Verdicker) sowie anorganische Verdicker, wie Betonite oder Kieselsäuren.

Das Aufschäumen im erfindungsgemäßen Verfahren geschieht durch mechanisches Rühren der Zusammensetzung bei hohen Drehzahlen durch Schütteln oder durch Entspannung eines Treibgases.

Das mechanische Aufschäumen kann mit beliebigen mechanischen Rühr-, Misch- und Dispergiertechniken erfolgen. In der Regel wird hierbei Luft eingetragen, aber auch Stickstoff und andere Gase können hierfür benutzt werden.

Der so erhaltene Schaum wird beim Aufschäumen oder unmittelbar danach auf ein Substrat aufgetragen oder in eine Form gegeben und getrocknet.

Der Auftrag kann beispielsweise durch Gießen oder Rakeln erfolgen, aber auch andere au sich bekannte Techniken sind möglich. Ein mehrschichtiger Auftrag mit zwischengeschalteten Trocknungsschritten ist grundsätzlich auch möglich.

Eine befriedigende Trocknungsgeschwindigkeit der Schäume wird bereits bei 20°C beobachtet, so dass eine Trocknung auf verletztem menschlichen oder tierischen Gewebe problemlos möglich ist. Für eine schnellere Trocknung und Fixierung der Schäume werden jedoch bevorzugt Temperaturen oberhalb von 30°C benutzt. Bei der Trocknung sollten jedoch Temperaturen von 200°C bevorzugt 150°C, besonders bevorzugt 130°C nicht überschritten werden, da es anderenfalls u.a. zu unerwünschter Vergilbung der Schäume kommen kann. Möglich ist auch eine zwei- oder mehrstufige Trocknung.

Die Trocknung erfolgt in der Regel unter Verwendung von an sich bekannten Heiz- und Trockenapparaten, wie (Umluft-) Trockenschränken, Heißluft oder IR-Strahlern.

Der Auftrag sowie die Trocknung können jeweils diskontinuierlich oder kontinuierlich durchgeführt werden, bevorzugt ist jedoch gänzlich kontinuierliches Verfahren.

Als Substrate geeignet sind Papiere oder Folien, die ein einfaches Ablösen der Wundauflage vor ihrem Einsatz zur Abdeckung einer verletzten Stelle ermöglichen. Ebenso kann als Substrat menschliches oder tierisches Gewebe wie Haut dienen, so dass eine direkte Verschließung einer verletzten Stelle durch eine in-situ hergestellte Wundauflage möglich ist.

Ein weiterer Gegenstand sind die nach dem erfindungsgemäßen Verfahren erhältlichen Wundauflagen.

Die Wundauflagen haben vor ihrer Trocknung typischerweise Schaumdichten von 50 bis 800 g/Liter, bevorzugt 100 bis 500 g/Liter, besonders bevorzugt 100 bis 250 g/Liter (Masse aller Einsatzstoffe [in g] bezogen auf das Schaumvolumen von einem Liter).

Die Wundauflagen besitzen nach ihrer Trocknung eine mikroporöse, offenporige Struktur mit miteinander kommunizierenden Zellen. Die Dichte der getrockneten Schäume liegt dabei typischerweise unterhalb von 0,4 g/cm³, bevorzugt ist sie geringer als 0,35 g/cm³, besonders bevorzugt 0,01 bis 0,3 g/cm³ und liegt ganz besonders bevorzugt bei 0,15 bis 0,3 g/cm³.

Das Absorptionsvermögen gegenüber physiologischer Salzlösung beträgt bei den Polyurethan-Schäumen typischerweise 100 bis 1500 %, bevorzugt 300 bis 1500 %, besonders bevorzugt 300 bis 800 % (Masse der aufgenommenen Flüssigkeit bezogen auf die Masse des trockenen Schaums; Bestimmung nach DIN EN 13726-1, Teil 3.2). Die Durchlässigkeit gegenüber Wasserdampf beträgt typischerweise 2000 bis 8000 g/24 h * m², bevorzugt 3000 bis 8000 g/24 h * m², besonders bevorzugt 3000 bis 5000 g/24 h *m² (Bestimmung nach DIN EN 13726-2, Teil 3.2).

Die Polyurethan-Schäume weisen eine gute mechanische Festigkeit und hohe Elastizität auf. Typischerweise sind die Werte für die maximale Spannung größer als 0,2 N/mm² und die maximale Dehnung ist größer als 250 %. Bevorzugt ist die maximale Spannung größer als 0,4 N/mm² und die Dehnung größer als 350 % (Bestimmung nach DIN 53504).

Die Wundauflagen haben nach dem Trocknen typischerweise eine Dicke von 0,1 mm bis 50 mm, bevorzugt 0,5 mm bis 20 mm, besonders bevorzugt 1 bis 10 mm, ganz besonders bevorzugt 1 bis 5 mm.

Die Wundauflagen können überdies mit weiteren Materialien verklebt, laminiert oder beschichtet werden, beispielsweise auf Basis von Hydrogelen, (semi-) permeablen Folien, Beschichtungen, Hydrokolloiden oder anderen Schäumen.

Sofern es zweckdienlich ist, kann im erfindungsgemäßen Verfahren ein Schritt zur Sterilisation erfolgen. Ebenso ist es grundsätzlich möglich, nach dem erfindungsgemäßen Verfahren erhältlichen Wundauflagen nach deren Herstellung zu sterilisieren. Zur Sterilisation kommen die dem Fachmann an sich bekannten Verfahren zum Einsatz, bei denen eine Sterilisation durch thermische Behandlung, chemische Stoffe wie Ethylenoxid oder Bestrahlung beispielsweise durch Gammabestrahlung erfolgt.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

Aufgrund der breiten Einsetzbarkeit des erfindungsgemäßen Verfahrens und der darüber zugänglichen Wundauflagen ist es grundsätzlich möglich, dieses in der industriellen Herstellung von Wundauflagen einzusetzen. Ebenso ist es aber auch möglich, dieses zur Herstellung z.B. von Sprühpflastem einzusetzen, wobei die Wundauflage durch direkten Auftrag der Zusammensetzung auf eine Wunde und gleichzeitiges Aufschäumen und anschließendes Trocknen gebildet wird.

Zur industriellen Herstellung von Wundauflagen wird die Polyurethan-Dispersion (I) mit Schaumhilfsmitteln der vorstehend genannten Art vermischt, danach mechanisch durch Eintrag eines Gases wie Luft aufgeschäumt. Dieser Schaum wird auf eine Unterlage aufgetragen und physikalisch getrocknet. Aufgrund höherer Produktivität erfolgt die Trocknung typischerweise bei erhöhten Temperaturen von 30 bis 200°C, bevorzugt 50 bis 150°C, besonders bevorzugt 60 bis 130°C. Bevorzugt ist überdies eine mindestens zweistufige Trocknung beginnend bei Temperaturen von 40 bis 80°C und mit anschließender weiterer Trocknung bei erhöhten Temperaturen von 80 bis 140°C. Die Trocknung erfolgt in der Regel unter Verwendung von an sich bekannten Heiz- und Trockenapparaten, z.B. (Umluft-) Trockenschränken. Der Auftrag sowie die Trocknung können jeweils diskontinuierlich oder kontinuierlich durchgeführt werden, bevorzugt ist jedoch gänzlich kontinuierliches Verfahren. Zur Sterilisation kann während oder nach dem Verfahren ein Sterilisationsschritt durch Bestrahlung oder Zugabe von geeigneten Substanzen erreicht werden.

Bei der Verwendung der erfindungswesentlichen Zusammensetzung bei der Herstellung eines Sprühpflasters wird die Polyurethan-Dispersion (I) mit einem Schaumhilfsmittel und einem Treibmittel formuliert, so dass beim Aufsprühen gleichzeitig eine Aufschäumung einsetzt. Zur Verfestigung wird der gebildete Schaum anschließend getrocknet, wobei Temperaturen von 20 bis 40°C bereits ausreichen. Unter Zuhilfenahme zusätzlicher Wärmequellen wie Fön oder IR-Rotlichtlampe ist allerdings auch eine forcierte Wärmetrocknung bis maximal 80°C möglich.

Die dabei eingesetzten Treibmittel sind an sich aus der Polyurethanchemie bekannt. So sind beispielsweise geeignet n-Butan, i-Butan und Propan sowie Mischungen aus diesen Kohlenwasserstoffen, ebenso geeignet ist beispielsweise aber auch Dimethylether. Bevorzugt wird eine Mischung aus n-Butan, i-Butan und Propan eingesetzt, wodurch die gewünschten, feinzelligen Schäume erhalten werden. Das Treibmittel oder das Treibmittelgemisch wird dabei typischerweise in einer Menge von 1 bis 50 Gew.-%, bevorzugt 5 bis 40 Gew.-% und besonders bevorzugt 5 bis 20 Gew.-% eingesetzt, wobei die Summe aus eingesetzter Polyurethan-Dispersion (I), Treibmittel (-gemisch) sowie gegebenenfalls eingesetzter Hilfs- und Zusatzstoffe (II) 100 Gew.-% ergibt. Die Bereitstellung der Sprühpflaster erfolgt bevorzugt in Sprühdosen. Neben dem Sprühen ist auch Gießen der Zusammensetzung möglich.

### Beispiele:

Sofern nicht abweichend gekennzeichnet, beziehen sich alle Prozentangaben auf das Gewicht.

Sofern nicht abweichend vermerkt, beziehen alle analytischen Messungen auf Temperaturen von 23°C.

Die Bestimmung der Festkörpergehalte erfolgte nach DIN-EN ISO 3251.

NCO-Gehalte wurden, wenn nicht ausdrücklich anders erwähnt, volumetrisch gemäß DINEN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt.

### Verwendete Substanzen und Abkürzungen:

- Diaminosulfonat:: NH₂-CH₂CH₂-NH-CH₂CH₂-SO₃Na (45 %ig in Wasser)
- Desmophen^{®} 2020/C2200:: Polycarbonatpolyol, OH-Zahl 56 mg KOH/g, zahlenmittleres Molekulargewicht 2000 g/mol (Bayer MaterialScience AG, Leverkusen, DE)
- PolyTHF^{®} 2000:: Polytetramethylenglykolpolyol, OH-Zahl 56 mg KOH/g, zahlenmittleres Molekulargewicht 2000 g/mol (BASF AG, Ludwigshafen, DE)
- PolyTHF^{®} 1000:: Polytetramethylenglykolpolyol, OH-Zahl 112 mg KOH/g, zahlenmittleres zahlenmittleres Molekulargewicht 1000 g/mol (BASF AG, Ludwigshafen, DE)
- Polyether LB 25:: monofunktioneller Polyether auf Ethylenoxid-/Propylenoxidbasis zahlenmittleres Molekulargewicht 2250 g/mol, OH-Zahl 25 mg KOH/g (Bayer MaterialScience AG, Leverkusen, DE)
- Stokal^{®} STA:: Schaumhilfsmittel auf Ammoniumstearat-Basis, Wirkstoffgehalt: 30 % (Bozzetto GmbH, Krefeld, DE)
- Stokal^{®} SR:: Schaumhilfsmittel auf Succinamat-Basis, Wirkstoffgehalt: ca. 34 % (Bozzetto GmbH, Krefeld, DE)
- Simulsol^{®} SL 26:: Alkylpolyglycosid auf Basis von Dodecylalkohol, ca. 52 %ig in Wasser, Seppic GmbH, Köln, DE

Die Bestimmung der mittleren Teilchengrößen (angegeben ist das Zahlenmittel) der Polyurethan-Dispersionen erfolgte mittels Laserkorrelations-Spektroskopie (Gerät: Malvern Zetasizer 1000, Malver Inst. Limited).

### Beispiel 1: Polyurethan-Dispersion 1

987,0 g PolyTHF^{®} 2000, 375,4 g PolyTHF^{®} 1000, 761,3 g Desmophen^{®} C2200 und 44,3 g Polyether LB 25 wurden in einer Standard-Rührapparatur auf 70°C aufgeheizt. Anschließend wurde bei 70°C innerhalb von 5 min ein Gemisch aus 237,0 g Hexamethylendiisocyanat und 313,2 g Isophorondiisocyanat zugegeben und bei 120°C gerührt, bis der theoretische NCO-Wert erreicht war. Das fertige Prepolymer wurde mit 4830 g Aceton gelöst und dabei auf 50°C abgekühlt und anschließend wurde eine Lösung aus 25,1 g Ethylendiamin, 116,5 g Isophorondiamin, 61,7 g Diaminosulfonat und 1030 g Wasser innerhalb von 10 min zudosiert. Die Nachrührzeit betrug 10 min. Dann wurde durch Zugabe von 1250 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 61 % |
| Partikelgröße (LKS): | 312 nm |
| Viskosität (Viskosimeter, 23°C): | 241 mPas |
| pH (23°C): | 6,02 |

### Beispiel 2: Polyurethan-Dispersion 2

223,7 g PolyTHF^{®} 2000, 85,1 g PolyTHF^{®} 1000, 172,6 g Desmophen^{®} C2200 und 10,0 g Polyether LB 25 wurden in einer Standard-Rührapparatur auf 70°C aufgeheizt. Anschließend wurde bei 70°C innerhalb von 5 min ein Gemisch aus 53,7 g Hexamethylendiisocyanat und 71,0 g Isophorondiisocyanat zugegeben und bei 120°C gerührt, bis der theoretische NCO-Wert erreicht war. Das fertige Prepolymer wurde mit 1005 g Aceton gelöst und dabei auf 50°C abgekühlt und anschließend wurde eine Lösung aus 5,70 Ethylendianin, 26,4 g Isophorondiamin, 9,18 g Diaminosulfonat und 249,2 g Wasser innerhalb von 10 min zudosiert. Die Nachrührzeit betrug 10 min. Dann wurde durch Zugabe von 216 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 63% |
| Partikelgröße (LKS): | 495 nm |
| Viskosität (Viskosimeter, 23°C): | 133 mPas |
| pH (23°C): | 6,92 |

### Beispiel 3: Polyurethan-Dispersion 3

987,0 g PolyTHF^{®} 2000, 375,4 g PolyTHF^{®} 1000, 761,3 g Desmophen^{®} C2200 und 44,3 g Polyether LB 25 wurden in einer Standard-Rührapparatur auf 70°C aufgeheizt. Anschließend wurde bei 70°C innerhalb von 5 min ein Gemisch aus 237,0 g Hexamethylendiisocyanat und 313,2 g Isophorondiisocyanat zugegeben und bei 120°C gerührt, bis der theoretische NCO-Wert erreicht war. Das fertige Prepolymer wurde mit 4830 g Aceton gelöst und dabei auf 50°C abgekühlt und anschließend wurde eine Lösung aus 36,9 g 1,4-Diaminobutan, 116,5 g Isophorondiamin, 61,7 g Diaminosulfonat und 1076 g Wasser innerhalb von 10 min zudosiert. Die Nachrührzeit betrug 10 min. Dann wurde durch Zugabe von 1210 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 59% |
| Partikelgröße (LKS): | 350 nm |
| Viskosität (Viskosimeter, 23°C): | 126 mPas |
| pH (23°C): | 7,07 |

### Beispiel 4: Polyurethan-Dispersion 4

201,3 g PolyTHF^{®} 2000, 76,6 g PolyTHF^{®} 1000, 155,3 g Desmophen^{®} C2200, 2,50 g 1,4-Butandiol und 10,0 g Polyether LB 25 wurden in einer Standard-Rührapparatur auf 70°C aufgeheizt. Anschließend wurde bei 70°C innerhalb von 5 min ein Gemisch aus 53,7 g Hexamethylendiisocyanat und 71,0 g Isophorondiisocyanat zugegeben und bei 120°C gerührt, bis der theoretische NCO-Wert erreicht war. Das fertige Prepolymer wurde mit 1010 g Acetön gelöst und dabei auf 50°C abgekühlt und anschließend wurde eine Lösung aus 5,70 Ethylendiamin, 26,4 g Isophorondiamin, 14,0 g Diaminosulfonat und 250 g Wasser innerhalb von 10 min zudosiert. Die Nachrührzeit betrug 10 min. Dann wurde durch Zugabe von 243 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 62% |
| Partikelgröße (LKS): | 566 nm |
| Viskosität (Viskosimeter, 23°C): | 57 mPas |
| pH (23°C): | 6,64 |

### Beispiel 5: Polyurethan-Dispersion 5

201,3 g PolyTHF^{®} 2000, 76,6 g PolyTHF^{®} 1000, 155,3 g Desmophen^{®} C2200, 2,50 g Trimethylolpropan und 10,0 g Polyether LB 25 wurden in einer Standard-Rührapparatur auf 70°C aufgeheizt. Anschließend wurde bei 70°C innerhalb von 5 min ein Gemisch aus 53,7 g Hexamethylendiisocyanat und 71,0 g Isophorondiisocyanat zugegeben und bei 120°C gerührt, bis der theoretische NCO-Wert erreicht war. Das fertige Prepolymer wurde mit 1010 g Aceton gelöst und dabei auf 50°C abgekühlt und anschließend wurde eine Lösung aus 5,70 Ethylendiamin, 26,4 g Isophorondiamin, 14,0 g Diaminosulfonat und 250 g Wasser innerhalb von 10 min zudosiert. Die Nachrührzeit betrug 10 min. Dann wurde durch Zugabe von 293 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 56% |
| Partikelgröße (LKS): | 440 nm |
| Viskosität (Viskosimeter, 23°C): | 84 mPas |
| pH (23°C): | 6,91 |

### Beispiel 6: Polyurethan-Dispersion 6

1072 g PolyTHF^{®} 2000, 407,6 g PolyTHF^{®} 1000, 827 g Desmophen^{®} C2200 und 48,1 g Polyether LB 25 wurden in einer Standard-Rührapparatur auf 70°C aufgeheizt. Anschließend wurde bei 70°C innerhalb von 5 min ein Gemisch aus 257,4 g Hexamethylendiisocyanat und 340 g Isophorondiisocyanat zugegeben und bei 120°C gerührt, bis der theoretische NCO-Wert erreicht war. Das fertige Prepolymer wurde mit 4820 g Aceton gelöst und dabei auf 50°C abgekühlt und anschließend wurde eine Lösung aus 27,3 g Ethylendiamin, 126,5 g Isophorondiamin, 67,0 g Diaminosulfonat und 1090 g Wasser innerhalb von 10 min zudosiert. Die Nachrührzeit betrug 10 min. Dann wurde durch Zugabe von 1180 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 60% |
| Partikelgröße (LKS): | 312 nm |
| Viskosität (Viskosimeter, 23°C): | 286 mPas |
| pH (23°C): | 7,15 |

### Beispiel 7:

54 g einer nach Beispiel 2 hergestellten Polyurethan-Dispersion wurden mit 1,37 g Simulsol^{®} SL 26 vermischt und in einer geeigneten Aerosol-Dose mit 6 g einer Treibmittelmischung aus i-Butan/Propan/n-Butan versetzt. Nach dem Sprühen (ca. 1 cm Nassfilmdicke) und Trocknen (10 Min. bei 120 °C) wurde ein reinweißer, feinzelliger Schaum erhalten.

### Beispiel 8:

54 g einer nach Beispiel 2 hergestellten Polyurethan-Dispersion wurden mit 1,37 g Simulsol^{®} SL 26 vermischt und in einer geeigneten Aerosol-Dose mit 6 g Dimethylether versetzt. Nach dem Sprühen (ca. 1 cm Nassfilmdicke) und Trocknen (10 Min. bei 120 °C) wurde ein reinweißer, feinzelliger Schaum erhalten.

### Vergleichsbeispiel 1

### Polyurethan-Dispersion, nicht erfindungsgemäß (keine Sulfonat-Gruppen, sondern nur Hydrophilierung durch nicht-ionische Gruppen und Carboxylat-Gruppen)

Es wurde vorgegangen analog Beispiel 1, jedoch wurde das Diaminosulfonat äquimolar ersetzt durch eine carboxylatgruppenhaltige Komponente:
206,8 g PolyTHF^{®} 2000, 78,7 g PolyTHF^{®} 1000, 159,5 g Desmophen^{®} C2200 und 9,3 g Polyether LB 25 wurden in einer Standard-Rührapparatur auf 70°C aufgeheizt. Anschließend wurde bei 70°C innerhalb von 5 min ein Gemisch aus 49,7 g Hexamethylendiisocyanat und 65,6 g Isophorondiisocyanat zugegeben und bei 120°C gerührt, bis der theoretische NCO-Wert erreicht war. Das fertige Prepolymer wurde mit 1010 g Aceton gelöst und dabei auf 50°C abgekühlt und anschließend wurde eine Lösung aus 5,3 g Ethylendiamin, 24,4 g Isophorondiamin, 11,9 g KV 1386 (40 %ige wässrige Lösung des Natriumsalzes von N-(2-Aminoethyl)-β-alanin, BASF AG, Ludwigshafen, DE) und 204 g Wasser innerhalb von 10 min zudosiert. Die Nachrührzeit betrug 10 min. Dann wurde durch Zugabe von 235 g Wasser dispergiert. Es folgte die Entfernung des Lösemittels durch Destillation im Vakuum. Dabei mussten aufgrund der hohen Viskosität noch insgesamt 250 g Wasser zugesetzt werden

Die erhaltene weiße Dispersion hatte nachfolgenden Eigenschaften:

| | |
|---|---|
| Feststoffgehalt: | 47 % |
| Partikelgröße (LKS): | 918 nm |
| Viskosität (Viskosimeter, 23°C): | 162 mPas |
| pH (23°C): | 7,22 |

Aufgrund der vergleichsweise hohen mittleren Teilchengröße von > 900 nm war im Gegensatz zu den rein sulfonathydrophilierten Dispersionen eine innerhalb weniger Tage einsetzende Sedimentation zu beobachten, nach welcher eine Weiterverarbeitung zu Wundauflagen erschwert war.

### Vergleichsbeispiel 2:

### Polyurethan-Dispersion, nicht erfindungsgemäß (keine Sulfonat-Gruppen, sondern nur Hydrophilierung durch nicht-ionische Gruppen und Carboxylat-Gruppen)

Es wurde vorgegangen analog Vergleichsbeispiel 1, jedoch wurde die Menge der carboxylatgruppenhaltigen Hydrophilierungskomponente um 50 % erhöht (bei gleichem Grad der Kettenverlängerung).

206,8 g PolyTHF^{®} 2000, 78,7 g PolyTHF^{®} 1000, 159,5 g Desmophen^{®} C2200 und 9,3 g Polyether LB 25 wurden in einer Standard-Rührapparatur auf 70°C aufgeheizt. Anschließend wurde bei 70°C innerhalb von 5 min ein Gemisch aus 49,7 g Hexamethylendiisocyanat und 65,6 g Isophorondiisocyanat zugegeben und bei 120°C gerührt, bis der theoretische NCO-Wert erreicht war. Das fertige Prepolymer wurde mit 1010 g Aceton gelöst und dabei auf 50°C abgekühlt und anschließend wurde eine Lösung aus 5,3 g Ethylendiamin, 21,8 g Isophorondiamin, 17,9 g KV 1386 (40 %ige wässrige Lösung des Natriumsalzes von N-(2-Aminoethyl)-β-alanin, BASF AG, Ludwigshafen, DE) und 204 g Wasser innerhalb von 10 min zudosiert. Die Nachrührzeit betrug 10 min. Dann wurde durch Zugabe von 235 g Wasser dispergiert. Es folgte die Entfernung des Lösemittels durch Destillation im Vakuum.

Die erhaltene weiße Dispersion hatte nachfolgenden Eigenschaften:

| | |
|---|---|
| Feststoffgehalt: | 52,2 % |
| Partikelgröße (LKS): | 255 nm |
| Viskosität (Viskosimeter, 23°C): | 176 mPas |
| pH (23°C): | 8,31 |

Diese Polyurethan-Dispersion zeigt jetzt zwar im Unterschied zum Beispiel 7 eine geringere mittlere Teilchengröße, jedoch einen etwas höheren pH-Wert. Eine Weiterverarbeitung zu Wundauflagen war im Vergleich zu rein sulfonathydrophilierten Dispersionen deutlich schwieriger.

### Beispiele 9 bis 14: Schäume, hergestellt aus den Polyurethan-Dispersionen der Beispiele 1 bis 6

Die in der Tabelle 1 angegebenen Mengen der Polyurethan-Dispersionen, hergestellt wie in den Beispielen 1 bis 6 beschrieben, wurden mit den Schaumhilfsmitteln wie dort ebenso angegeben vermischt und unter Verwendung eines handelsüblichen Handrührgerätes (Rührer aus gebogenem Draht) auf 1 Liter Schaumvolumen aufgeschlagen. Danach wurden die Polyurethan-Schäume mittels eines Filmziehgerätes (Rakel) mit einer Spalthöhe von 4 mm auf siliconbeschichtetes Papier aufgezogen. In der Tabelle 1 sind ebenso die Trocknungsbedingungen der wie angegeben hergestellten Polyurethan-Schäume aufgeführt. Es wurden durchweg reinweiße Polyurethan-Schäume mit guten mechanischen Eigenschaften und einer feinen Porenstruktur erhalten.

**Tabelle 1**

| | Menge [g] | | | |
|---|---|---|---|---|
| Schaum Nr. | Polyurethan-Dispersion (Beispiel) | Stokal^{®} STA | Stokal^{®} SR | Aushärtung |
| 9a | 235,0 (1) | 4,2 | 5,6 | 2 h / 37°C |
| 9b | 235,0 (1) | 4,2 | 5,6 | 2 h / 37°C, 30 min / 110 °C |
| 10 | 235,0 (2) | 4,2 | 5,6 | 2 h / 37°C, 30 min / 120 °C |
| 11a | 235,0 (3) | 4,2 | 5,6 | 2 h / 37°C |
| 11b | 235,0 (3) | 4,2 | 5,6 | 2 h / 37°C, 30 min / 120 °C |
| 12a | 235,0 (4) | 4,2 | 5,6 | 2 h / 37°C |
| 12b | 235,0 (4) | 4,2 | 5,6 | 2 h / 37°C, 30 min / 120 °C |
| 13a | 235,0 (5) | 4,2 | 5,6 | 2 h / 37°C |
| 13b | 235,0 (5) | 4,2 | 5,6 | 2 h / 37°C, 30 min / 120 °C |
| 14 | 235,0 (6) | 4,2 | 5,6 | 2 h / 37°C, 30 min / 120 °C |

Wie der Tabelle 2 zu entnehmen ist, zeigten allen Polyurethan-Schäume eine sehr schnelle Aufnahme von Wasser, eine hohe Absorption von physiologischer Salzlösung ("Saugleistung bei freier Quellmöglichkeit"), eine sehr gute Wasserdampfdurchlässigkeit ("MVTR") und darüber hinaus eine gute mechanische Festigkeit, insbesondere auch nach Feuchtelagerung.

**Tabelle 2**

| Schaum Nr. | Aufnahmegeschwindigkeit¹⁾ [s] | Freie Saugleistung²⁾ [g/100 cm²] | MVTR³⁾ [g/m²*24 h] |
|---|---|---|---|
| 9a | nicht bestimmt | 23,1 | 4300 |
| 9b | nicht bestimmt | 19,2 | 5000 |
| 10 | 3 | 28,4 | 4700 |
| 11a | 4 | 20,6 | 4300 |
| 11b | 14 | 18,3 | 4300 |
| 12a | 4 | 24,7 | 4800 |
| 12b | 7 | 26,7 | 4500 |
| 13a | 5 | 25,5 | 4800 |
| 13b | 7 | 23,1 | 4100 |
| 14 | 4 | 21,3 | nicht bestimmt |

| | | | |
|---|---|---|---|
| ¹⁾ Zeit bis zum vollständigen Eindringen eines Tropfens (destilliertes Wasser) in den Schaum; ²⁾ Absorption physiologischer Salzlösung bestimmt nach DIN EN 13726-1, Teil 3.2 (5 anstelle von 9 Prüfmustern); ³⁾ "moisture vapour transition rate" (Wasserdampfdurchlässigkeit) bestimmt nach DIN EN 13726-2, Teil 3.2 | | | |

## Patentansprüche

1. Verfahren zur Herstellung von Wundauflagen, bei dem Zusammensetzungen enthaltend anionisch hydrophilierte, wässrige Polyurethan-Dispersionen (I) aufgeschäumt und physikalisch ohne chemische Vernetzung getrocknet werden, **dadurch gekennzeichnet, dass** die Polyurethan-Dispersionen (I) zur anionischen Hydrophilierung ausschließlich Sulfonatgruppen enthalten und die Dispersionen (I) Feststoffgehalte von 55 bis 65 Gew.-% bezogen auf das darin enthaltene Polyurethan aufweisen.

2. Verfahren gemäß Anspruch 1, **dadurch gekennzeichnet, dass** die Sulfonatgruppen als Gegenionen Alkalimetallkationen besitzen.

3. Verfahren gemäß einem der Ansprüche 1 bis 2, **dadurch gekennzeichnet, dass** Polyurethan-Dispersionen (I) 0,1 bis 15 Milliequivalente pro 100 g Festharz an anionischen oder potentiell anionischen Gruppen bezogen auf Festharz aufweisen.

4. Verfahren gemäß einem der Ansprüche 1 bis 3, **dadurch gekennzeichnet, dass** die Dispersionen (I) erhältlich sind, indem
A) isocyanatfunktionelle Prepolymere aus
A1) organischen Polyisocyanaten
A2) polymeren Polyolen mit zahlenmittleren Molekulargewichten von 400 bis 8000 g/mol und OH-Funktionalitäten von 1,5 bis 6 und
A3) gegebenenfalls hydroxyfunktionellen Verbindungen mit Molekulargewichten von 62 bis 399 g/mol und
A4) gegebenenfalls isocyanatreaktiven, anionischen oder potentiell anionischen und gegebenenfalls nichtionischen Hydrophilierungsmitteln, hergestellt, und
B) deren freie NCO-Gruppen dann ganz oder teilweise
B1) gegebenenfalls mit aminofunktionellen Verbindungen mit Molekulargewichten von 32 bis 400 g/mol und
B2) mit aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden.

5. Verfahren gemäß einem der Ansprüche 1 bis 4, **dadurch gekennzeichnet, dass** die aufzuschäumenden Zusammensetzungen als Hilfs- und Zusatzstoffe (II) Fettsäureamide, Sulfosuccinamide, Kohlenwasserstoffsulfonate, bzw. -sulfate, Alkylpolyglycoside und/oder Fettsäuresalze als Schaumbildner und -stabilisatoren enthalten.

6. Verfahren gemäß Anspruch 5, **dadurch gekennzeichnet, dass** als Schaumbildner und -stabilisatoren Mischungen aus Sulfosuccinamiden und Ammoniumstearaten verwendet werden, wobei diese 70 bis 50 Gew.-% an Sulfosuccinamiden enthalten.

7. Wundauflagen erhältlich nach einem Verfahren gemäß einem der Ansprüche 1 bis 6.

8. Wundauflagen gemäß Anspruch 7, **dadurch gekennzeichnet, dass** diese eine mikroporöse, offenporige Struktur aufweisen und eine Dichte in getrocknetem Zustand von unterhalb 0,4 g/cm³ aufweisen.

9. Wundauflagen gemäß Anspruch 7 oder 8, **dadurch gekennzeichnet, dass** diese ein Absorptionsvermögen gegenüber physiologischer Salzlösung von 100 bis 1500 % (Masse der aufgenommenen Flüssigkeit bezogen auf die Masse des trockenen Schaums; Bestimmung nach DIN EN 13726-1, Teil 3.2) und eine Durchlässigkeit gegenüber Wasserdampf von 2000 bis 8000 g/24 h * m² besitzen (Bestimmung nach DIN EN 13726-2, Teil 3.2).

10. Wundauflagen gemäß einem der Ansprüche 7 bis 9, **dadurch gekennzeichnet, dass** diese auch einen Wirkstoff enthalten.

## Claims

1. Process for producing wound contact materials which comprises compositions containing anionically hydrophilicized, aqueous polyurethane dispersions (I) being frothed and physically dried without chemical crosslinking, **characterized in that** the polyurethane dispersions (I) are anionically hydrophilicized by sulfonate groups only and the dispersions (I) have solids contents in the range from 55% to 65% by weight based on the polyurethane present therein.

2. Process according to Claim 1, **characterized in that** the sulfonate groups have alkali metal cations as counter-ions.

3. Process according to either of Claims 1 and 2, **characterized in that** polyurethane dispersions (I) comprise 0.1 to 15 milliequivalents per 100 g of solid resin of anionic or potentially anionic groups based on solid resin.

4. Process according to any one of Claims 1 to 3, **characterized in that** the dispersions (I) are obtainable by
A) isocyanate-functional prepolymers being produced from
A1) organic polyisocyanates
A2) polymeric polyols having number-average molecular weights in the range from 400 to 8000 g/mol and OH functionalities in the range from 1.5 to 6 and
A3) optionally hydroxyl-functional compounds having molecular weights in the range from 62 to 399 g/mol and
A4) optionally isocyanate-reactive, anionic or potentially anionic and optionally nonionic hydrophilicizing agents
and
B) its free NCO groups then being wholly or partly reacted
B1) optionally with amino-functional compounds having molecular weights in the range from 32 to 400 g/mol and
B2) with amino-functional, anionic or potentially anionic hydrophilicizing agents
by chain extension, and the prepolymers being dispersed in water before, during or after step B).

5. Process according to any one of Claims 1 to 4, **characterized in that** the compositions to be frothed contain, as auxiliary and additive materials (II), fatty acid amides, sulfosuccinamides, hydrocarbyl sulfonates or sulfates, alkylpolyglycosides and/or fatty acid salts as foam formers and stabilizers.

6. Process according to Claim 5, **characterized in that** mixtures of sulfosuccinamides and ammonium stearates are used as foam formers and stabilizers, these mixtures containing 70% to 50% by weight of sulfosuccinamides.

7. Wound contact materials obtainable by a process according to any one of Claims 1 to 6.

8. Wound contact materials according to Claim 7, **characterized in that** they have a microporous, opencell structure and a density of below 0.4 g/cm³ in the dried state.

9. Wound contact materials according to Claim 7 or 8, **characterized in that** they have a DIN EN 13726-1 Part 3.2 physiological saline absorbency in the range from 100 to 1500% (mass of liquid taken up, based on the mass of dry foam) and a DIN EN 13726-2 Part 3.2 water vapor transmission rate in the range from 2000 to 8000 g/24 h * m².

10. Wound contact materials according to any one of Claims 7 to 9, **characterized in that** they also contain an active component.

## Revendications

1. Procédé pour la fabrication de pansements, dans lequel on transforme en mousse des compositions contenant des dispersions aqueuses de polyuréthane (I) hydrophilisées par voie anionique et on les sèche physiquement sans réticulation chimique, **caractérisé en ce que** pour l'hydrophilisation anionique les dispersions de polyuréthane (I) contiennent exclusivement des groupes sulfonate et les dispersions (I) présentent des teneurs en matière solide de 55 à 65 % en poids, par rapport au polyuréthane contenu dans celles-ci.

2. Procédé selon la revendication 1, **caractérisé en ce que** les groupes sulfonate comportent comme ions opposés des cations de métaux alcalins.

3. Procédé selon la revendication 1 ou 2, **caractérisé en ce que** les dispersions de polyuréthane (I) comportent par rapport à la résine solide 0,1 à 15 milliéquivalents pour 100 g de résine solide de groupes anioniques ou potentiellement anioniques.

4. Procédé selon l'une quelconque des revendications 1 à 3, **caractérisé en ce que** les dispersions (I) peuvent être obtenues par préparation
A) de prépolymères à fonction isocyanate à partir
A1) de polyisocyanates organiques
A2) de polyols polymères ayant des masses moléculaires moyennes en nombre de 400 à 8 000 g/mole et des fonctionnalités OH de 1,5 à 6 et
A3) éventuellement de composés à fonction hydroxy ayant des masses moléculaires de 62 à 399 g/mole et
A4) éventuellement d'agents d'hydrophilisation anioniques ou potentiellement anioniques et éventuellement non ioniques, réactifs avec des isocyanates,
et
B) ensuite mise en réaction de leurs groupes NCO libres, en totalité ou en partie
B1) éventuellement avec des composés à fonction amino, ayant des masses moléculaires de 32 à 400 g/mole et
B2) avec des agents d'hydrophilisation anioniques ou potentiellement anioniques, à fonction amino,
avec prolongement de chaîne, et avant, pendant ou après l'étape B) on disperse les prépolymères dans de l'eau.

5. Procédé selon l'une quelconque des revendications 1 à 4, **caractérisé en ce que** les compositions à transformer en mousse contiennent comme adjuvants et additifs (II) des amides d'acides gras, des sulfosuccinamides, des sulfonates ou sulfates d'hydrocarbures, des alkylpolyglycosides et/ou des sels d'acides gras en tant que générateurs et stabilisants de mousse.

6. Procédé selon la revendication 5, **caractérisé en ce qu'**on utilise comme générateurs et stabilisants de mousse des mélanges de sulfosuccinamides et stéarates d'ammonium, qui contiennent de 70 à 50 % en poids de sulfosuccinamides.

7. Pansements pouvant être obtenus conformément à un procédé selon l'une quelconque des revendications 1 à 6.

8. Pansements selon la revendication 7, **caractérisés en ce qu'**ils présentent une structure microporeuse à pores ouverts et ont une densité à l'état sec inférieure à 0,4 g/cm³.

9. Pansements selon la revendication 7 ou 8, **caractérisés en ce qu'**ils présentent un pouvoir d'absorption vis-à-vis d'une solution saline physiologique de 100 à 1 500 % (masse du liquide absorbé par rapport à la masse de la mousse sèche ; détermination selon DIN EN 13726-1, section 3.2) et une perméabilité à la vapeur d'eau de 2 000 à 8 000 g/24 h*m² (détermination selon DIN EN 13726-2, section 3.2).

10. Pansements selon l'une quelconque des revendications 7 à 9, **caractérisés en ce qu'**ils contiennent également une substance active.
